# EUROPEAN PATENT APPLICATION

(11) **EP 4 613 846 A1**
(43) Date of publication of application: **10.09.2025**
(21) Application number: 25151118.4
(22) Date of filing: 10.01.2025
(51) Int. Cl.: C12N 5/00, C12N 5/079

(54) **NERVOUS SYSTEM CELL-CONTAINING SPHEROID, METHOD FOR PRODUCING NERVOUS SYSTEM CELL-CONTAINING SPHEROID, AND METHOD FOR EVALUATING TEST SUBSTANCE**

(30) Priority: 06.03.2024 JP 2024034265; 26.09.2024 JP 2024167397
(71) Applicant: Ricoh Company, Ltd., Tokyo 143-8555 (JP)
(72) Inventor: IJICHI, Rie, Tokyo 143-8555 (JP)
(74) Representative: Marks & Clerk LLP

(57) **Abstract**

What is provided is a nervous system cell-containing spheroid having suitable drug responsiveness. There are provided a nervous system cell-containing spheroid including at least one kind of nerve cells and nervous system cells other than the nerve cells, in which the proportion of the volume of one cell layer positioned at an outer edge of the spheroid to the volume of the entire spheroid is 15% to 30%; a method for producing the nervous system cell-containing spheroid; and a method for evaluating a test substance, the method including a step of incubating a nervous system cell-containing spheroid in the presence of a test substance, and a step of evaluating an effect of the test substance on the nervous system cell-containing spheroid, in which the nervous system cell-containing spheroid includes at least one kind of nerve cells and nervous system cells other than the nerve cells, and the proportion of the volume of one cell layer positioned at an outer edge of the spheroid to the volume of the entire spheroid is 15% to 30%.

## Description

### BACKGROUND OF THE INVENTION

### Field of the Invention

The present invention relates to a nervous system cell-containing spheroid, a method for producing the nervous system cell-containing spheroid, and a method for evaluating a test substance. The present application claims priority on Japanese Patent Application No. 2024-034265, filed on March 6, 2024, and Japanese Patent Application No. 2024-167397, filed on September 26, 2024, the contents of which are incorporated herein by references.

### Description of Related Art

In research and development for drug discovery, in-vitro experimental systems using cells are often used. For example, also in a drug screening for a cranial nerve disease or a toxicity screening for a cerebral nervous system, primary cultured cells, cells differentiated from pluripotent stem cells such as embryonic stem cells (ES cells) and induced pluripotent stem cells (iPS cells), immortalized cells, cancer cells, or the like are used.

For nerve cells derived from the iPS cells among those, patient-derived cells can be used, whereby it is possible to reflect a genetic background derived from a disease, and the cells have been attracting attention as cells that can construct a model with high extrapolation.

For example, Patent Document 1 (JP2021-185906A) describes a nervous system cell-containing spheroid and a method for producing the same. It is also described that a functional variation of the spheroid can be decreased by setting a ratio of nerve cells to astrocytes constituting the spheroid to 1:1.

### SUMMARY OF THE INVENTION

As described later in Examples, the present inventors have found that a threedimensionally cultured (3D-cultured) nervous system cell-containing spheroid has lower drug responsiveness, as compared with a two-dimensionally cultured (2D-cultured) nervous system cell. An object of the present invention is to provide a nervous system cell-containing spheroid having suitable drug responsiveness.

The nervous system cell-containing spheroid according to the present invention includes at least one kind of nerve cells and nervous system cells other than the nerve cells, and the proportion of the volume of one cell layer positioned at an outer edge of the spheroid to the volume of the entire spheroid is 15% to 30%.

According to the present invention, it is possible to provide a nervous system cell-containing spheroid having suitable drug responsiveness.

### BRIEF DESCRIPTION OF THE DRAWING

FIG. 1 is a graph showing the results of a calcium assay in Experimental Example 1.
FIG. 2 is a graph showing the results of a calcium assay in Experimental Example 2.
FIG. 3 is a graph showing the results of a calcium assay in Experimental Example 3.
FIG. 4 is a graph showing the results of a calcium assay in Experimental Example 4.
FIG. 5 is a graph showing the results of a calcium assay in Experimental Example 5.
FIG. 6 is a graph showing the results of a calcium assay in Experimental Example 6.
FIG. 7 is a graph showing the results of a calcium assay in Experimental Example 7.
FIG. 8 is a graph showing the results of a calcium assay in Experimental Example 8.
FIG. 9 is a graph showing the results of a calcium assay in Experimental Example 9.
FIG. 10 is a representative image of a spheroid imaged in Experimental Example 10.

### DETAILED DESCRIPTION OF THE INVENTION

### [Nervous System Cell-Containing Spheroid]

In one embodiment, the present invention provides a nervous system cell-containing spheroid. The nervous system cell-containing spheroid of the present embodiment includes at least one kind of nerve cells and nervous system cells other than the nerve cells, and the proportion of the volume of one cell layer positioned at an outer edge of the spheroid to the volume of the entire spheroid is 15% to 30%.

As described later in Examples, the nervous system cell-containing spheroid of the present embodiment has suitable drug responsiveness, as compared with a nervous system cell-containing spheroid in the related art. In the present specification, the suitable drug responsiveness means, for example, that a drug concentration-dependent change in a signal indicating the drug responsiveness can be detected with high sensitivity, that the drug concentration-dependent change in a signal indicating the drug responsiveness can be detected with sensitivity equal to or higher than that of 2D-cultured nerve cells, that the drug responsiveness is improved, as compared with a nervous system cell-containing spheroid in a related art (a spheroid in which the proportion of the volume of one cell layer positioned at an outer edge of the spheroid to the volume of the entire spheroid to the volume of the entire spheroid is less than 15%), and that the drug responsiveness is high. The nervous system cell-containing spheroid of the present embodiment can be said to be a spheroid for evaluation of drug responsiveness.

In the nervous system cell-containing spheroid of the present embodiment, the proportion of the volume of one cell layer positioned at an outer edge of the spheroid to the volume of the entire spheroid is 15% to 30%. That is, in the nervous system cell-containing spheroid of the present embodiment, the proportion calculated by Expression (1) is 15% to 30%, and may be 20% to 25%. On the other hand, as described later in Examples, in a nervous system cell-containing spheroid in the related art, the proportion calculated by the Expression (1) is less than 15%. The present inventors have found that in a case where the volume of one cell layer positioned at an outer edge of the spheroid is 15% or more with respect to the volume of the entire spheroid, the drug responsiveness for a drug in contact with the spheroid increases. In addition, as described later in Examples, the nervous system cell-containing spheroid, in which the proportion calculated by Expression (1) is more than 30%, has a small volume as a spheroid in the first place and has a small number of cells constituting the spheroid itself, and thus, a signal showing the drug responsiveness may be small and may not be detected. From these, the present inventors have found that in a case where the proportion of the volume of one cell layer positioned at an outer edge of the spheroid to the volume of the entire spheroid is set to 15% to 30%, and more preferably about 20% to 25%, the drug responsiveness of the spheroid is suitable. Proportion (%) = Volume of one cell layer positioned at outer edge of spheroid/Volume of entire spheroid × 100

Here, the volume of the entire spheroid can be calculated based on the diameter of the spheroid obtained by observing the spheroid with an optical microscope or the like. The diameter of the spheroid may be determined, for example, as a diameter of a circle C having the same area as the two-dimensional projected area of the spheroid. That is, the volume of the entire spheroid may be determined as the volume of a sphere S1 having the same diameter as the circle C having the same area as the two-dimensional projected area of the spheroid.

In addition, the volume of one cell layer positioned at an outer edge of the spheroid may be determined, for example, by assuming that the diameter of one cell is 10 µm, and subtracting the volume of a sphere S2 having a radius 10 µm smaller than the radius of the sphere S1 from the volume of the sphere S1. Here, for example, it is considered that the diameter of one cell can be assumed to be 10 µm, based on the photographs of the nervous system cells described on the homepage of Elixirgen Scientific, Inc., and the like.

In one embodiment, the proportion calculated by Expression (1) can be adjusted by adjusting the total number of cells constituting the spheroid. As described later, the proportion calculated by Expression (1) may vary depending on the type of the cells constituting the spheroid (volume per one cell). As described later in Examples, the spheroid in which the proportion calculated by Expression (1) is 15% to 30% can be said to be a spheroid in which the total number of cells is more than 2,000 and less than 16,000 (a spheroid in which the number of cells constituting the spheroids is more than 2,000 and less than 16,000). Therefore, in a case of being defined by the number of cells constituting the spheroid, the total number of cells of the nervous system cell-containing spheroid according to the present embodiment is more than 2,000 and less than 16,000, preferably 3,000 to 12,000, and more preferably 4,000 to 8,000.

In one embodiment, the spheroid in which the proportion calculated by Expression (1) is 15% to 30% can be said to be a spheroid having a diameter of 150 to 450 µm. Therefore, in a case of being defined by the diameter of the spheroid, the nervous system cell-containing spheroid according to the present embodiment has a diameter of 150 to 450 µm, preferably 192 to 394 µm, and more preferably 225 to 325 µm.

As described later in Examples, it can be said that the spheroid in which the proportion calculated by Expression (1) is 15% to 30% has a cell density of 0.35 to 1.1 cells/10³ µm³. Therefore, in a case of being defined by the cell density of the spheroid, the nervous system cell-containing spheroid of the present embodiment has a cell density of 0.35 to 1.1 cells/10³ µm³, preferably 0.38 to 0.81 cells/10³ µm³, and more preferably 0.45 to 0.67 cells/10³ µm³. Here, the cell density of the spheroid can be calculated, for example, by dividing the number of cells constituting the spheroid by the volume of the sphere S1.

In a case where the number of cells constituting the spheroid is small, voids are likely to be formed between the cells, and as the number of cells constituting the spheroid is large, the cells are densely accumulated and the voids between the cells tend to be small. In addition, even with the numbers of cells constituting the spheroid being the same, in the production of the spheroid, in a case where the culture period after seeding the cells is 2 to 3 days, the void between the cells tends to be larger, and in a case where the culture period is 7 days or more, the void between the cells tends to be smaller.

It is preferable that the spheroid of the present embodiment has a small void between the cells. In one embodiment, the spheroid cultured during a culture period of, for example, about 30 days to 50 days for after seeding the cells can be suitably used for the evaluation of the drug responsiveness.

In the present specification, the nervous system cell means a cell constituting a nervous system, and preferably means a cell constituting a central nervous system of a vertebrate. Examples of the nervous system cell include nerve cells as well as glial cells such as astrocytes, oligodendrocytes, and microglia.

The nervous system cell-containing spheroid of the present embodiment contains at least one kind of nerve cells and nervous system cells other than the nerve cells. It is preferable that the nervous system cells other than the nerve cells include astrocytes. The nervous system cell-containing spheroid of the present embodiment further include nervous system cells other than the nerve cells and the astrocytes.

In the nervous system cell-containing spheroid of the present embodiment, the proportion of the number of the nerve cells to the number of the astrocytes (the number of the nerve cells/the number of the astrocytes) is preferably 1. In the present specification, the proportion of the number of the nerve cells to the number of the astrocytes of 1 includes a case where there is a variation of about ± 20% in the proportion. That is, a value of the number of the nerve cells/the number of the astrocytes does not need to be exactly 1, and may be, for example, 0.8 to 1.2 or 0.9 to 1.1.

The present inventors have previously found that a spheroid in which the number of the nerve cells:the number of the astrocytes is about 1:1 tends to have a smaller functional variation, as compared with a spheroid in which the number of the nerve cells:the number of the astrocytes is about 2:1 and a spheroid in which the number of the nerve cells:the number of the astrocytes is about 4:1.

The function of the nervous system cell-containing spheroid refers to a value measured by evaluating an activity of the nervous system cell-containing spheroid by some assay. For example, the number of times of spontaneous oscillation per predetermined time, as measured by subjecting the nervous system cell-containing spheroid to a calcium assay, may be used. The calcium assay will be described later. Alternatively, it may be responsiveness to a drug. Examples of the responsiveness to a drug include a change in spontaneous oscillation in the presence of the drug, a change in expression level of a specific gene, and the like, which are not particularly limited.

The small functional variation may be, for example, a case where the variation calculated according to the following calculation method is less than 20%, and may be, for example, less than 15%, for example, less than 10%.

### (Calculation Method)

A plurality of nervous system cell-containing spheroids between a plurality of lots or within the same lot are each subjected to a calcium assay, the number of times of spontaneous oscillation per 10 minutes is measured, an average value of the number of times of spontaneous oscillation, and a standard deviation value thereof are calculated, and a variation thereof is calculated according to Expression (2). Variation (%) = Standard deviation of number of times of spontaneous oscillation/Average value of number of times of spontaneous oscillation × 100

The number of the nerve cells and the number of the astrocytes included in the spheroid may be considered to be equal to the number of the nerve cells and the number of the astrocytes, which have been mixed in the step of producing the nervous system cell-containing spheroid.

Alternatively, for example, the number of cells may be measured and obtained by subjecting the spheroids to fluorescence immunostaining, staining each of the nerve cells and the astrocytes, and observing the cells with a fluorescence microscope. Examples of a marker of the nerve cell include MAP2, Tubulin beta3, NeuN, 160 kDa Neurofilament, 200 kDa Neurofilament, NSE, PSD93, and PSD95. Examples of a marker of the astrocyte include GFAP, S100β, Cx43, EAAT1, EAAT2, Glutamine synthetase, andALDHIL1.

Alternatively, the number of the nerve cells and the astrocytes in the spheroid may not be measured as the number of cells, and the total fluorescence intensity of each stained cell may be regarded as a value corresponding to the number of cells to calculate the proportion of the number of the nerve cells to the number of the astrocytes.

Alternatively, the proportion of the number of the nerve cells to the number of the astrocytes may be calculated by regarding the area or volume of each stained cell on the microscopic image as a value corresponding to the number of cells.

The nervous system cell-containing spheroid of the present embodiment is usually accommodated in a container. The form of the container is not particularly limited, and examples thereof include a tube and a multiwell plate. Examples of the multiwell plate include a 24-well plate, a 48-well plate, a 96-well plate, a 384-well plate, and a 1,536-well plate.

The shape, the volume, the material, the color, and the like of the wells of the multiwell plate are not particularly limited, and can be appropriately selected according to the purpose.

The shape of the well is not particularly limited as long as the well can accommodate the spheroid, and can be appropriately selected according to the purpose. Examples of the shape include a flat bottom, a round bottom, a U bottom, and a V bottom.

The volume of the well is not particularly limited, and can be appropriately selected according to the purpose, and for example, in consideration of the amount of a reagent used in a general evaluation method, the volume of the well may be 5 to 1,000 µL, 30 to 300 µL, or 50 to 200 µL.

Examples of the color of the multiwell plate include being transparent, translucent, colored, and completely light-shielding. In addition, in a case of performing evaluation of an optical system, from the viewpoint of suppressing an interference between adjacent wells, a container in which the bottom surface part is transparent and the side surface part is colored is preferable.

The material of the multiwell plate can be appropriately selected according to the purpose, and examples thereof include acrylic materials such as polyethylene terephthalate (PET), polystyrene (PS), polycarbonate (PC), triacetyl cellulose (TAC), polyimide (PI), nylon (Ny), low-density polyethylene (LDPE), medium-density polyethylene (MDPE), vinyl chloride, vinylidene chloride, polyphenylene sulfide, polyethersulfone, polyethylene naphthalate, polypropylene, and urethane acrylate; organic materials such as cellulose and polydimethylsiloxane (PDMS); and inorganic materials such as glass and ceramics.

In order to prevent the spheroids from adhering to a bottom surface part or a side surface part of the multiwell plate, it is preferable that a surface of the multiwell plate is subjected to a low adsorption treatment. Examples of the treatment include coating with a polymer having a phosphorylcholine group, a covalent bonding treatment of a neutrally charged hydrophilic gel, and coating with a synthetic polymer having the same structure as a polar group of phosphatidylcholine. The treatment is not limited to these treatments as long as it is a treatment for suppressing cell adhesion, and can be appropriately selected.

The nervous system cell-containing spheroid of the present embodiment may be accommodated in a container, in which the container has a multiwell plate shape and contains one nervous system cell-containing spheroid in each well. Such a container is convenient because it can be used as it is for various assays.

### [Method for Producing Nervous System Cell-Containing Spheroid]

In one embodiment, the present invention provides a method for producing a nervous system cell-containing spheroid. The production method of the present embodiment is a production method including a step of mixing at least one kind of nerve cells after cell lineage determination and nervous system cells other than the nerve cells at a predetermined proportion to form a spheroid, in which the proportion of the volume of one cell layer positioned at an outer edge of the spheroid to the volume of the entire spheroid is adjusted to 15% to 30% in the step.

In the present specification, the expression, "the cell after cell lineage determination", is a cell whose terminally differentiated cell type has been determined in a case where undifferentiated cells are differentiated, and means a cell having differentiation potency, in which it has been determined which specific cell type it will be terminally differentiated into, or a cell which has already completed the terminal differentiation. Therefore, the "nervous system cell after cell lineage determination" may be a differentiated nervous system cell or may be a stem cell destined to be differentiated into a nervous system cell. The differentiated nervous system cell may be a primary cell extracted from a living body or may be a nervous system cell induced to be differentiated from a stem cell. In addition, the stem cell may be a pluripotent stem cell or a neural progenitor cell.

Examples of the pluripotent stem cell include embryonic stem cells (ES cells), induced pluripotent stem cells (iPS cells), and nuclear transfer embryonic stem cells (ntES cells). Among these, the iPS cells are preferable as the pluripotent stem cells.

The iPS cells may be derived from healthy individuals or patients with various nervous system diseases. In addition, the cells may also be various kinds of gene-edited cells, such as cells modified through gene editing to carry genes that cause or are risk factors for various nervous system diseases.

In a case where the iPS cells are derived from the patients with various nervous system diseases, these cells can be used to construct models of the nervous system diseases. The nervous system diseases are not particularly limited, but examples thereof include neurodegenerative diseases, autism, epilepsy, attention-deficit hyperactivity disorder (ADHD), schizophrenia, and bipolar disorder. Examples of the neurodegenerative diseases include Alzheimer's disease, Parkinson's disease, and amyotrophic lateral sclerosis.

The animal species from which the nerve cells are derived is not particularly limited, and examples thereof include primates such as humans and monkeys, mammals such as rabbits and dogs, ungulates such as pigs, sheep, horses, and cows, and rodents such as mice, rats, guinea pigs, and hamsters. Among these, the humans are preferable.

As described above, examples of the nervous system cell include glial cells such as astrocytes, oligodendrocytes, and microglia, in addition to the nerve cells.

The nerve cells can be broadly classified into, for example, peripheral nerves and central nerves. Examples of the peripheral nerves include sensory nerve cells, motor nerve cells, and autonomic nerve cells. Examples of the central nerves include interneurons and projection neurons. Examples of the projection neurons include cortical neurons, hippocampal neurons, and amygdala neurons. In addition, the central nerve cells can be broadly classified into excitatory neurons and inhibitory neurons. Examples thereof include glutamatergic neurons which are mainly responsible for excitatory transmission in the central nervous system, and γ-aminobutyric acid (GABA)-ergic neurons which are mainly responsible for inhibitory transmission.

Other examples of the neurons that release neuromodulators include cholinergic neurons, dopaminergic neurons, noradrenergic neurons, serotonergic neurons, and histaminergic neurons.

In addition, astrocytes, oligodendrocytes, microglia, and the like may be primary cultured cells or cells induced to be differentiated from stem cells. In a case where the cells induced to be differentiated from stem cells are used, it is preferable to use a nervous system cell after cell lineage determination, that is, a nervous system cell after it is determined to be differentiated into an astrocyte, an oligodendrocyte, a microglia, or the like.

The nervous system cell after the cell lineage determination may be a pluripotent stem cell which has been subjected to a differentiation induction treatment into a nerve cell. Examples of the differentiation induction treatment include introduction of a specific transcription factor into a pluripotent stem cell. Specific examples of such a cell include cells of Quick-Neuron^{™} series of Elixirgen Scientific Inc. These cells are pluripotent stem cells that have been subjected to a differentiation induction treatment into a nervous system cell, and are differentiated into functionally mature nervous system cells in about 10 days.

After mixing a plurality of types of nervous system cells after cell lineage determination at a predetermined proportion, the mixture is seeded in an appropriate cell culture container such as a plate for producing a spheroid, and cultured to form a spheroid. The period for incubating the cell culture container can be appropriately set according to the purpose, but is preferably at least a sufficient time for the nervous system cell to mature to an extent that a function thereof can be evaluated.

For example, in a case where the pluripotent stem cell which has been subjected to a differentiation induction treatment into a nervous system cell is used as a nervous system cell after the cell lineage determination, the period can be, for example, 20 days or more, for example, 30 days or more, for example, 40 days or more, for example, 50 days or more, for example, 60 days or more, or for example, 70 days or more from a time point when the pluripotent stem cells after the differentiation induction treatment into a plurality of types of nervous system cells are mixed and seeded. In one embodiment, a spheroid of, for example, about 30 days to 50 days from a time point when the pluripotent stem cells after the differentiation induction treatment into a plurality of types of nervous system cells are mixed and seeded can be suitably used for the evaluation of the drug responsiveness.

As the culture medium, a culture medium obtained by adding necessary components to a basal culture medium can be used. Examples of the basal culture medium include BrianPhys (STEMCELL Technologies Inc.), Neurobasal (Thermo Fisher Scientific, Inc.), Neurobasal Plus (Thermo Fisher Scientific, Inc.), Dulbecco's Modified Eagle's Medium (DMEM), a Ham F12 medium (Ham's Nutrient Mixture F12), a D-MEM/F12 medium, a McCoy's 5A medium, an Eagle's MEM medium (Eagle's Minimum Essential Medium, EMEM), an αMEM medium (alpha Modified Eagle's Medium Essential Medium, αMEM), an MEM medium (Minimum Essential Medium), an RPMI1640 (Roswell Park Memorial Institute-1640) medium, an Iscove's Modified Dulbecco's Medium (IMDM), an MCDB131 medium, a William Medium E, an IPL41 medium, a Fischer's medium, an M199 medium, a High Performance Medium 199, a StemPro34 (Thermo Fisher Scientific, Inc.), X-VIVO 10 (Chembrex), X-VIVO 15 (Chembrex), HPGM (Chembrex), StemSpan H3000 (Stem Cell Technologies), StemSpan SFEM (Stem Cell Technologies), Stemline II (Sigma-Aldrich), QBSF-60 (Quality Biological), StemPro hESC SFM (Thermo Fisher Scientific, Inc.), an Essentials (registered trademark) medium (Thermo Fisher Scientific, Inc.), an mTeSR1 or mTeSR2 medium (Stem Cell Technologies), Repro FF or Repro FF2 (Reprocell), a PSGro hESC/iPSC medium (System Biosciences), a NutriStem (registered trademark) medium (Biological Industries), a CSTI-7 medium (Cell Science Laboratory), a MesenPRO RS medium (Thermo Fisher Scientific, Inc.), an MF-Medium (registered trademark) mesenchymal stem cell growth medium (Toyobo Co., Ltd.), Sf-900II (Thermo Fisher Scientific, Inc.), and Opti-Pro (Thermo Fisher Scientific, Inc.). These may be used alone or a mixture of two or more kinds thereof may be used.

In addition, examples of the additive to be added to the basal culture medium include those generally used for nerve cell culture, examples of which include an SM1 supplement (STEMCELL Technologies Inc.), an N2 supplement A (STEMCELL Technologies Inc.), a rat astrocyte culture supernatant (FUJIFILM Wako Pure Chemical Corporation), a human astrocyte culture supernatant (ScienCell Research Laboratories, Inc.), Component N (Elixirgen Scientific, Inc.), Component G2 (Elixirgen Scientific, Inc.), Component P (Elixirgen Scientific, Inc.), N2 Supplement (Thermo Fisher Scientific, Inc.), iCell Neural Supplement B (Cellular Dynamics International), iCell Neuvous System Supplement, and B-27 Plus (Thermo Fisher Scientific, Inc.).

In the step of forming a spheroid, the adjustment of the proportion of the volume of one cell layer positioned at an outer edge of the spheroid to the volume of the entire spheroid to 15% to 30% means that the proportion calculated by Expression (1) is adjusted to 15% to 30%, and more preferably 20% to 25%, and specifically, this can be realized by adjusting the number of the nervous system cells to be mixed.

As described later in Examples, the spheroid in which the proportion calculated by Expression (1) is 15% to 30% can be said to be a spheroid in which the total number of cells is more than 2,000 and less than 16,000 (a spheroid in which the number of cells constituting the spheroids is more than 2,000 and less than 16,000). Therefore, the nervous system cell-containing spheroid in which the proportion calculated by Expression (1) is 15% to 30% can be produced by setting the number of the nervous system cells to be mixed to more than 2,000 and less than 16,000, preferably 3,000 to 12,000, and more preferably 4,000 to 8,000 to form the spheroid.

As described later in Examples, it can be said that the spheroid in which the proportion calculated by Expression (1) is 15% to 30% has a cell density of 0.35 to 1.1 cells/10³ µm³. Therefore, the production method of the present embodiment can also be said to be a method for producing a nervous system cell-containing spheroid, in which the cell density is 0.35 to 1.1 cells/10³ µm³. The cell density is preferably 0.38 to 0.81 cells/10³ µm³, and more preferably 0.45 to 0.67 cells/10³ µm³.

It is considered that in a case where the types of cells are different, the spheroid sizes are different even in a case where the numbers of cells are the same. Therefore, it is necessary to specify an appropriate number (range) of cells for each cell to be used, in which the volume ratio of one cell layer is 15% to 30%.

Specifically, first, a plurality of spheroids in which the number of cells to be used is gradually increased (for example, the total number of cells is 20,000, 16,000, 12,000, 8,000, 4,000, 2,000, or the like) are formed. Then, "the volume ratio of one cell layer/the volume of the entire cell" for each cell number is calculated, based on Expression (1). Then, the number of cells (range) at which the value is 15% to 30% is calculated. Hereinafter, production of the spheroid is performed using a predetermined number of cells according to the type of cells to be used. In this manner, it is possible to produce a spheroid having suitable drug sensitivity (drug responsiveness).

In the production method of the present embodiment, it is preferable that the nervous system cells other than the nerve cells include astrocytes. The nervous system cells may further include nervous system cells other than the nerve cells and the astrocytes.

In the production method of the present embodiment, the proportion of the number of the nerve cells to the number of the astrocytes (the number of the nerve cells/the number of the astrocytes) is preferably 1. The fact that the proportion of the number of the nerve cells to the number of the astrocytes is 1 is the same as described above.

### [Method for Evaluating Test Substance]

In one embodiment, the present invention provides a method for evaluating a test substance. The evaluation method of the present embodiment includes a step of incubating a nervous system cell-containing spheroid in the presence of the test substance, and a step of evaluating an effect of the test substance on the nervous system cell-containing spheroid, in which the nervous system cell-containing spheroid includes at least one kind of nerve cells and nervous system cells other than the nerve cells, and the proportion of the volume of one cell layer positioned at an outer edge of the spheroid to the volume of the entire spheroid is 15% to 30%.

The evaluation method of the present embodiment is a method for evaluating a test substance using the above-described nervous system cell-containing spheroid. The above-described nervous system cell-containing spheroid has suitable drug responsiveness, as compared with a nervous system cell-containing spheroid in the related art. Therefore, the test substance can be appropriately evaluated by the evaluation method of the present embodiment.

The evaluation method of the present embodiment can also be said to be a method for screening a drug. The test substance is not particularly limited, and examples thereof include a natural compound library, a synthetic compound library, an existing drug library, and a metabolite library.

The method for evaluating an influence of the test substance on the nervous system cell-containing spheroid is not particularly limited, and examples thereof include a calcium assay, RNA-seq, immunostaining of the entire spheroid, immunostaining of a thin section of the spheroid, and microelectrode array (MEA) analysis.

Here, the calcium assay will be described. In the calcium assay, first, a nervous system cell-containing spheroid is prepared. For example, a mixture of any number of the nerve cells and the astrocytes is seeded on a plate for producing a spheroid, and the cells are cultured for 3 to 8 weeks while appropriately exchanging the culture medium with a culture medium suitable for culturing the nerve cells.

Subsequently, the spheroid is transferred to a plate suitable for optical detection, and a calcium-sensitive fluorescent dye is added to the culture medium. The calcium-sensitive fluorescent dye is a substance that emits fluorescence in a case of being taken up into cells and binding to calcium ions. As the calcium-sensitive fluorescent dye, for example, Cal-520 (AAT Bioquest, Inc.), Cal-520AM (AAT Bioquest, Inc.), Fluo4 (AAT Bioquest, Inc.), Calcium-6 (Molecular Devices, LLC), or EarlyTox Cardiotoxicity Kit (Molecular Devices, LLC) can be appropriately used.

Subsequently, an excitation wavelength/a fluorescence wavelength suitable for the calcium-sensitive fluorescent dye used is set in a device capable of detecting the fluorescence, and the measurement is continuously performed for any period. Examples of the device that can be used include a fluorescence plate reader, a functional drug screening system (FDSS, Hamamatsu Photonics K. K.), and a fluorometric imaging plate reader (FLIPR, Molecular Devices, LLC). For example, the measurement may be performed for 20 minutes at an interval of 0.6 seconds.

A nerve cell that is performing a nerve activity spontaneously repeats the uptake and the release of calcium ions. Therefore, in a case where a calcium assay is performed using the nervous system cell-containing spheroid, the fluorescence intensity changes periodically and is measured as a spontaneous oscillation.

In the evaluation method of the present embodiment, it is preferable that the nervous system cells other than the nerve cells constituting the nervous system cell-containing spheroid include astrocytes. The nervous system cells may further include nervous system cells other than the nerve cells and the astrocytes.

In the evaluation method of the present embodiment, the proportion of the number of the nerve cells to the number of the astrocytes constituting the nervous system cell-containing spheroid (the number of the nerve cells/the number of the astrocytes) is preferably 1. The fact that the proportion of the number of the nerve cells to the number of the astrocytes is 1 is the same as described above.

### Examples

Next, the present invention will be described in more detail with reference to Examples, but is not limited to the following Examples.

### [Experimental Example 1]

### (Examination of Drug Responsiveness of 2D-Cultured nerve cells and 3D Spheroid-Cultured Nerve Cells)

### «2D Culture of Nerve Cells»

Human iPSC-derived nerve cells (Excitatory Neurons, Elixirgen Scientific, Inc.) and human-derived primary cultured astrocytes (ScienCell Research Laboratories, Inc.) were seeded in a 384-well plate so that the number of cells per well was 10,000 for the nerve cells and 2,500 for the astrocytes. As the culture medium, Neurobasal Plus (Thermo Fisher Scientific, Inc.) to which B-27 Plus Supplement (Thermo Fisher Scientific, Inc.), Glutamax (Thermo Fisher Scientific, Inc.), 200 µM ascorbic acid (FUJIFILM Wako Pure Chemical Corporation), and a 10% Neuron Culture medium (FUJIFILM Wako Pure Chemical Corporation) had been added was used. Subsequently, the cells were cultured for 6 weeks and a calcium assay which will be described later was performed.

### <3D Spheroid Culture of Nerve Cells>

Human iPSC-derived nerve cells (Excitatory Neurons, Elixirgen Scientific, Inc.) and human-derived primary cultured astrocytes (ScienCell Research Laboratories, Inc.) were mixed so that the number of cells per well was 8,000 for the nerve cells and 8,000 for the astrocytes, and seeded on a plate for producing a spheroid. As the culture medium, Neurobasal Plus (Thermo Fisher Scientific, Inc.) to which B-27 Plus Supplement (Thermo Fisher Scientific, Inc.), Glutamax (Thermo Fisher Scientific, Inc.), 200 µM ascorbic acid (FUJIFILM Wako Pure Chemical Corporation), and a 10% Neuron Culture medium (FUJIFILM Wako Pure Chemical Corporation) had been added was used. Subsequently, the cells were cultured for 6 weeks and a calcium assay which will be described later was performed.

### <Calcium Assay>

A calcium assay was performed in the presence of a drug to examine the drug responsiveness of the 2D-cultured nerve cells and the 3D spheroid-cultured nerve cells. Chlorpromazine was used as the drug.

In the case of the 2D-cultured nerve cells, a calcium-sensitive fluorescent dye (Cal-520AM, AAT Bioquest, Inc.) and a drug were added to the culture medium, and the fluorescence was measured at intervals of 0.5 seconds for 20 minutes using a fluorescence plate reader to measure the calcium spikes.

In the case of the 3D spheroid-cultured nerve cells, each spheroid was transferred to a plate for measurement, a calcium-sensitive fluorescent dye (Cal-520AM, (AAT Bioquest, Inc.)), Pluronic F-127 (AAT Bioquest, Inc.), and the drug were added to the culture medium, and the fluorescence was measured at intervals of 0.5 seconds for 20 minutes using a fluorescence plate reader to measure the calcium spikes.

### «Results»

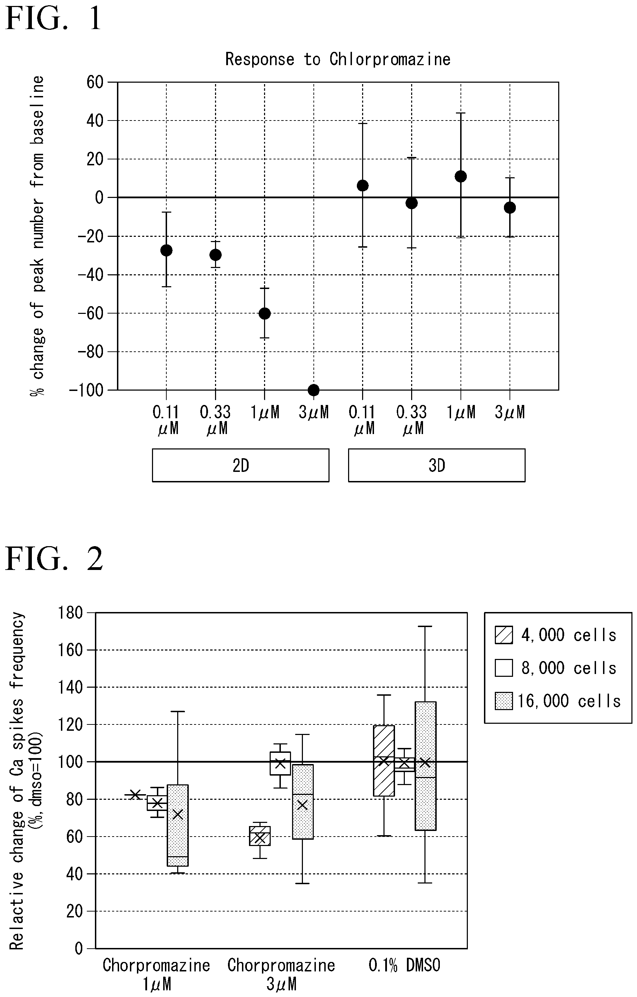

FIG. 1 is a graph showing the measurement results. The values in the graph indicate the mean value ± the standard deviation. The horizontal axis of the graph indicates the concentration of chlorpromazine in the culture medium, and the vertical axis of the graph indicates the proportion of the change in number of the calcium spikes. In addition, "2D" indicates the results of the 2D-cultured nerve cells, and "3D" indicates the results of the 3D spheroid-cultured nerve cells.

By setting the proportion of the change in number of the calcium spikes in a case where dimethyl sulfoxide (DMSO) used as a solvent for the drug was added to 0%, in a case where the number of the calcium spikes increased due to the addition of the drug, the value is shown to be positive, and in a case where the number of the calcium spikes decreased due to the addition of the drug, the value is shown to be negative.

As a result, in the 2D-cultured nerve cells, the number of the calcium spikes decreased in a drug concentration-dependent manner, and the calcium spikes disappeared at a concentration of 3 µM. On the other hand, in the 3D spheroid-cultured nerve cells, no change in number of the calcium spikes was observed at any drug concentration.

These results show that the drug responsiveness of the 3D spheroid-cultured nerve cells is lower than that of the 2D-cultured nerve cells.

### [Experimental Example 2]

### (Examination 1 of Drug Responsiveness of 3D Spheroid-Cultured Nerve Cells)

A calcium assay was performed in the presence of a drug, using spheroids with different numbers of cells, and the drug responsiveness of the nerve cells was examined.

Human iPSC-derived nerve cells (Excitatory Neurons, Elixirgen Scientific, Inc.) and human-derived primary cultured astrocytes (ScienCell Research Laboratories, Inc.) were mixed so that the number of cells per well was 8,000 for the nerve cells and 8,000 for the astrocytes, 4,000 for the nerve cells and 4,000 for the astrocytes, or 2,000 for the nerve cells and 2,000 for the astrocytes, and seeded on a plate for producing a spheroid. As the culture medium, Neurobasal Plus (Thermo Fisher Scientific, Inc.) to which B-27 Plus Supplement (Thermo Fisher Scientific, Inc.), Glutamax (Thermo Fisher Scientific, Inc.), 200 µM ascorbic acid (FUJIFILM Wako Pure Chemical Corporation), and a 10% Neuron Culture medium (FUJIFILM Wako Pure Chemical Corporation) had been added was used. Subsequently, the cells were cultured for 5 weeks, and a calcium assay was performed in the same manner as in Experimental Example 1. Chlorpromazine was used as the drug. The expected drug response is a decrease in number of calcium spikes in a case where chlorpromazine is applied.

FIG. 2 is a graph showing the measurement results. The values in the graph indicate the mean value ± the standard deviation. The horizontal axis of the graph indicates the concentration of chlorpromazine in the culture medium, and the vertical axis of the graph indicates a relative value of the number of the calcium spikes in a case where dimethyl sulfoxide (DMSO) used as a solvent for the drug was added, with the number of the calcium spikes set to 100%. A case where the number of the calcium spikes has increased due to the addition of the drug shows a numerical value more than 100%, and a case where the number of the calcium spikes has decreased shows a numerical value smaller than 100%. 0% indicates a state in which the calcium spikes completely disappear.

In FIG. 2, "16,000 cells" indicates the results of the spheroids produced by mixing 8,000 nerve cells and 8,000 astrocytes, "8,000 cells" indicates the results of the spheroids produced by mixing 4,000 nerve cells and 4,000 astrocytes, and "4,000 cells" indicates the results of the spheroids produced by mixing 2,000 nerve cells and 2,000 astrocytes.

As a result, in a case of evaluating the drug responsiveness to 1 µM and 3 µM of chlorpromazine, the 16,000 cells and 8,000 cells did not show a concentration-dependent response, whereas the 4,000 cells showed a concentration-dependent response.

These results show that the drug responsiveness varies depending on the number of cells constituting the spheroid, and the drug responsiveness tends to be suitable in a case where the number of cells is low.

### [Experimental Example 3]

### (Examination 2 of Drug Responsiveness of 3D Spheroid-Cultured Nerve Cells)

A calcium assay was performed using spheroids having different cell numbers in the same manner as in Experimental Example 2, except that the drug was changed to bicuculine, and the drug responsiveness of the nerve cells was examined. The expected drug response is an increase in number of calcium spikes in a case where bicuculine is applied.

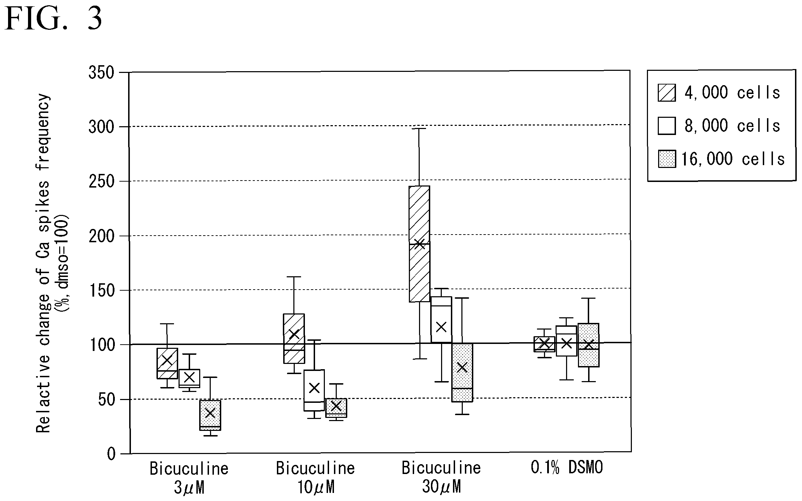

FIG. 3 is a graph showing the measurement results. The values in the graph indicate the mean value ± the standard deviation. The horizontal axis of the graph indicates the concentration of bicuculine in the culture medium, and the vertical axis of the graph indicates a relative value of the number of the calcium spikes in a case where dimethyl sulfoxide (DMSO) used as a solvent for the drug was added, with the number of the calcium spikes set to 100%. A case where the number of the calcium spikes has increased due to the addition of the drug shows a numerical value more than 100%, and a case where the number of the calcium spikes has decreased shows a numerical value smaller than 100%. 0% indicates a state in which the calcium spikes completely disappear.

In FIG. 3, "16,000 cells" indicates the results of the spheroids produced by mixing 8,000 nerve cells and 8,000 astrocytes, "8,000 cells" indicates the results of the spheroids produced by mixing 4,000 nerve cells and 4,000 astrocytes, and "4,000 cells" indicates the results of the spheroids produced by mixing 2,000 nerve cells and 2,000 astrocytes.

As a result, an increase in the number of the calcium spikes was observed in a concentration-dependent manner at 4,000 cells. In 8,000 cells, a significant response was not observed in about 4,000 cells, but a tendency of an increase in the number of the calcium spikes was observed at 30 µM. At a cell density of 16,000 cells, an increase in the number of the calcium spikes was not observed at any concentration.

These results further support that the drug responsiveness varies depending on the number of cells constituting the spheroid, and the drug responsiveness tends to be suitable in a case where the number of cells is low.

### [Experimental Example 4]

### (Examination 3 of Drug Responsiveness of 3D Spheroid-Cultured Nerve Cells)

A calcium assay was performed in the same manner as in Experimental Example 2, except that the drug was changed to GABA and the number of cells constituting the spheroid was changed to 16,000 or 4,000, and the drug responsiveness of the nerve cells was examined. The expected drug response is a decrease in number of calcium spikes in a case where GABA is applied.

Human iPSC-derived nerve cells (Excitatory Neurons, Elixirgen Scientific, Inc.) and human-derived primary cultured astrocytes (ScienCell Research Laboratories, Inc.) were mixed so that the number of cells per well was 4,000 nerve cells and 4,000 astrocytes, or 2,000 nerve cells and 2,000 astrocytes, and seeded on a plate for producing a spheroid. As the culture medium, Neurobasal Plus (Thermo Fisher Scientific, Inc.) to which B-27 Plus Supplement (Thermo Fisher Scientific, Inc.), Glutamax (Thermo Fisher Scientific, Inc.), 200 µM ascorbic acid (FUJIFILM Wako Pure Chemical Corporation), and a 10% Neuron Culture medium (FUJIFILM Wako Pure Chemical Corporation) had been added was used. Subsequently, the cells were cultured for 5 weeks, and a calcium assay was performed in the same manner as in Experimental Example 2. GABA was used as the drug. The expected drug response is a decrease in number of calcium spikes in a case where GABA is applied.

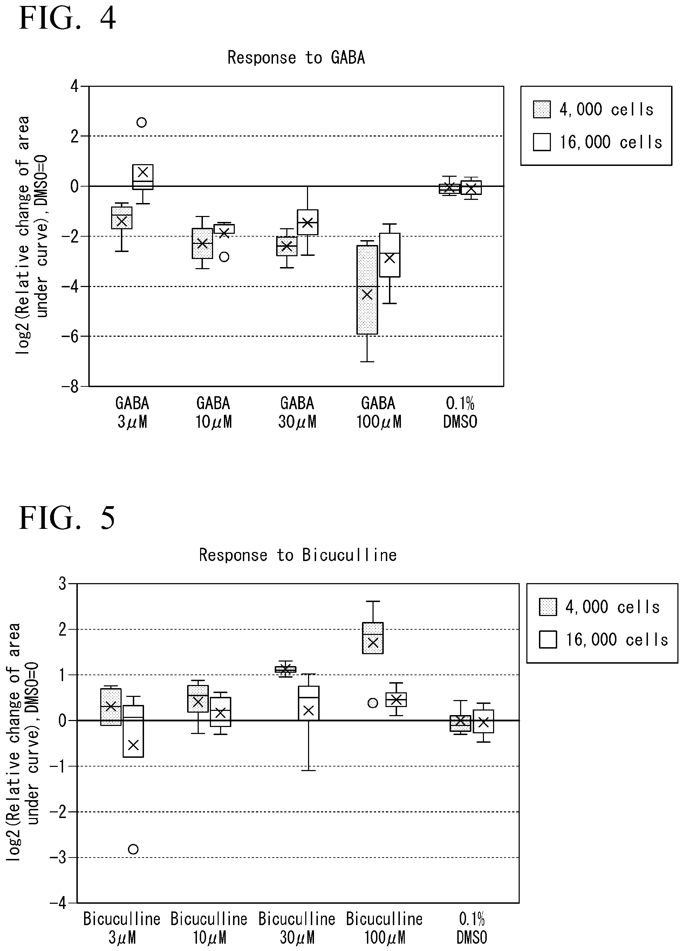

FIG. 4 is a graph showing the measurement results. The values in the graph indicate the mean value ± the standard deviation. The horizontal axis of the graph indicates the concentration of GABA in the culture medium, and the vertical axis of the graph indicates the relative value (log2) of a change in number of the calcium spikes. The response observed in a case where DMSO, which is a solvent of the drug, is added is indicated as 0 (no change), a positive numerical value is indicated in a case where the number of the calcium spikes increased due to the addition of the drug, and a negative numerical value is indicated in a case where the number of the calcium spikes decreased.

In FIG. 4, "16,000 cells" indicates the results of the spheroids produced by mixing 8,000 nerve cells and 8,000 astrocytes, and "4,000 cells" indicates the results of the spheroids produced by mixing 2,000 nerve cells and 2,000 astrocytes.

As a result, it was shown that the action was exhibited at a lower concentration and a stronger action was exhibited at 4,000 cells in a concentration-dependent manner, and suitable results of the drug sensitivity were obtained, as compared with 16,000 cells.

These results further support that the drug responsiveness varies depending on the number of cells constituting the spheroid, and the drug responsiveness tends to be suitable in a case where the number of cells is low.

### [Experimental Example 5]

### (Examination 4 of Drug Responsiveness of 3D Spheroid-Cultured Nerve Cells)

A calcium assay was performed in the same manner as in Experimental Example 4, except that the drug was changed to bicuculine, and the drug responsiveness of the nerve cells was examined. The expected drug response is an increase in number of calcium spikes in a case where bicuculine is applied.

FIG. 5 is a graph showing the measurement results. The values in the graph indicate the mean value ± the standard deviation. The horizontal axis of the graph indicates the concentration of bicuculine in the culture medium, and the vertical axis of the graph indicates the relative value (log2) of a change in number of the calcium spikes. The response observed in a case where DMSO, which is a solvent of the drug, is added is indicated as 0 (no change), a positive numerical value is indicated in a case where the number of the calcium spikes increased due to the addition of the drug, and a negative numerical value is indicated in a case where the number of the calcium spikes decreased.

In FIG. 5, "16,000 cells" indicates the results of the spheroids produced by mixing 8,000 nerve cells and 8,000 astrocytes, and "4,000 cells" indicates the results of the spheroids produced by mixing 2,000 nerve cells and 2,000 astrocytes.

As a result, no drug response was observed at 16,000 cells, whereas drug responsiveness was exhibited in a concentration-dependent manner at 4,000 cells.

These results further support that the drug responsiveness varies depending on the number of cells constituting the spheroid, and the drug responsiveness tends to be suitable in a case where the number of cells is low.

### [Experimental Example 6]

### (Examination 5 of Drug Responsiveness of 3D Spheroid-Cultured Nerve Cells)

A calcium assay was performed in the same manner as in Experimental Example 4, except that the drug was changed to 6-cyano-7-nitroquinoxaline-2,3-dione (CNQX), and the drug responsiveness of the nerve cells was examined. The expected drug response is a decrease in number of calcium spikes in a case where CNQX is applied.

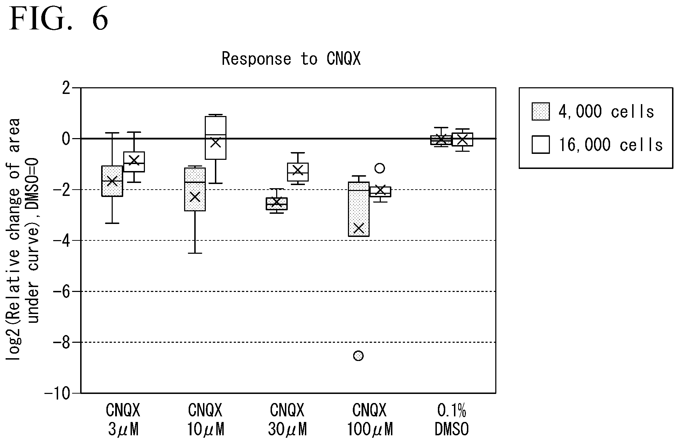

FIG. 6 is a graph showing the measurement results. The values in the graph indicate the mean value ± the standard deviation. The horizontal axis of the graph indicates the concentration of CNQX in the culture medium, and the vertical axis of the graph indicates the relative value (log2) of a change in number of the calcium spikes. The response observed in a case where DMSO, which is a solvent of the drug, is added is indicated as 0 (no change), a positive numerical value is indicated in a case where the number of the calcium spikes increased due to the addition of the drug, and a negative numerical value is indicated in a case where the number of the calcium spikes decreased.

In FIG. 6, "16,000 cells" indicates the results of the spheroids produced by mixing 8,000 nerve cells and 8,000 astrocytes, and "4,000 cells" indicates the results of the spheroids produced by mixing 2,000 nerve cells and 2,000 astrocytes.

As a result, it was shown that the action was exhibited at a lower concentration and a stronger action was exhibited at 4,000 cells in a concentration-dependent manner, and suitable results of the drug sensitivity were obtained, as compared with 16,000 cells.

These results further support that the drug responsiveness varies depending on the number of cells constituting the spheroid, and the drug responsiveness tends to be suitable in a case where the number of cells is low.

### [Experimental Example 7]

### (Examination 6 of Drug Responsiveness of 3D Spheroid-Cultured Nerve Cells)

A calcium assay was performed in the same manner as in Experimental Example 4, except that the drug was changed to chlorpromazine, and the drug responsiveness of the nerve cells was examined. The expected drug response is a decrease in the number of calcium spikes in a case where chlorpromazine is applied.

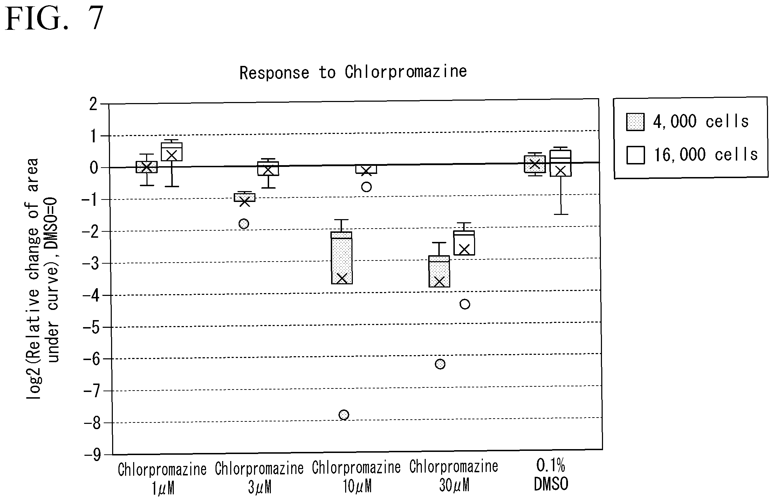

FIG. 7 is a graph showing the measurement results. The values in the graph indicate the mean value ± the standard deviation. The horizontal axis of the graph indicates the concentration of chlorpromazine in the culture medium, and the vertical axis of the graph indicates the relative value (log2) of a change in number of the calcium spikes. The response observed in a case where DMSO, which is a solvent of the drug, is added is indicated as 0 (no change), a positive numerical value is indicated in a case where the number of the calcium spikes increased due to the addition of the drug, and a negative numerical value is indicated in a case where the number of the calcium spikes decreased.

In FIG. 7, "16,000 cells" indicates the results of the spheroids produced by mixing 8,000 nerve cells and 8,000 astrocytes, and "4,000 cells" indicates the results of the spheroids produced by mixing 2,000 nerve cells and 2,000 astrocytes.

As a result, it was shown that the action was exhibited at a lower concentration and a stronger action was exhibited at 4,000 cells in a concentration-dependent manner, and suitable results of the drug sensitivity were obtained, as compared with 16,000 cells.

These results further support that the drug responsiveness varies depending on the number of cells constituting the spheroid, and the drug responsiveness tends to be suitable in a case where the number of cells is low.

### [Experimental Example 8]

### (Examination 7 of Drug Responsiveness of 3D Spheroid-Cultured Nerve Cells)

A calcium assay was performed in the same manner as in Experimental Example 4, except that the drug was changed to dopamine, and the drug responsiveness of the nerve cells was examined. The expected drug response is a decrease in number of calcium spikes in a case where dopamine is applied.

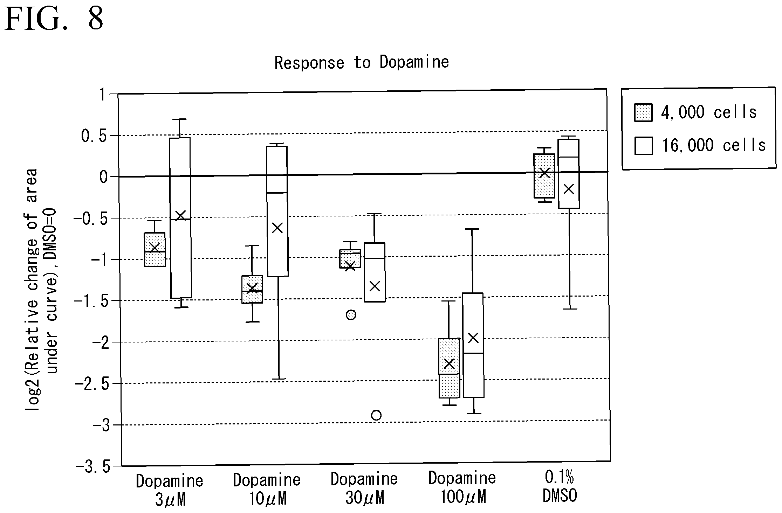

FIG. 8 is a graph showing the measurement results. The values in the graph indicate the mean value ± the standard deviation. The horizontal axis of the graph indicates the concentration of dopamine in the culture medium, and the vertical axis of the graph indicates the relative value (log2) of a change in number of the calcium spikes. The response observed in a case where DMSO, which is a solvent of the drug, is added is indicated as 0 (no change), a positive numerical value is indicated in a case where the number of the calcium spikes increased due to the addition of the drug, and a negative numerical value is indicated in a case where the number of the calcium spikes decreased.

In FIG. 8, "16,000 cells" indicates the results of the spheroids produced by mixing 8,000 nerve cells and 8,000 astrocytes, and "4,000 cells" indicates the results of the spheroids produced by mixing 2,000 nerve cells and 2,000 astrocytes.

As a result, it was shown that the action was exhibited at a lower concentration and a stronger action was exhibited at 4,000 cells in a concentration-dependent manner, and suitable results of the drug sensitivity were obtained, as compared with 16,000 cells.

These results further support that the drug responsiveness varies depending on the number of cells constituting the spheroid, and the drug responsiveness tends to be suitable in a case where the number of cells is low.

### [Experimental Example 9]

### (Examination 8 of Drug Responsiveness of 3D Spheroid-Cultured Nerve Cells)

A calcium assay was performed in the same manner as in Experimental Example 4, except that the drug was changed to carbamazepine, and the drug responsiveness of the nerve cells was examined. The expected drug response is a decrease in the number of calcium spikes in a case where carbamazepine is applied.

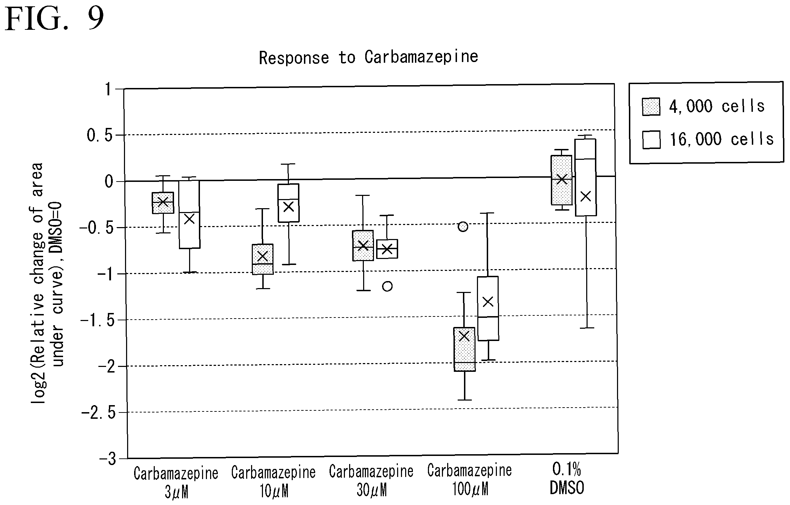

FIG. 9 is a graph showing the measurement results. The values in the graph indicate the mean value ± the standard deviation. The horizontal axis of the graph indicates the concentration of carbamazepine in the culture medium, and the vertical axis of the graph indicates the relative value (log2) of a change in number of the calcium spikes. The response observed in a case where DMSO, which is a solvent of the drug, is added is indicated as 0 (no change), a positive numerical value is indicated in a case where the number of the calcium spikes increased due to the addition of the drug, and a negative numerical value is indicated in a case where the number of the calcium spikes decreased.

In FIG. 9, "16,000 cells" indicates the results of the spheroids produced by mixing 8,000 nerve cells and 8,000 astrocytes, and "4,000 cells" indicates the results of the spheroids produced by mixing 2,000 nerve cells and 2,000 astrocytes.

As a result, it was shown that the action was exhibited at a lower concentration and a stronger action was exhibited at 4,000 cells in a concentration-dependent manner, and suitable results of the drug sensitivity were obtained, as compared with 16,000 cells.

These results further support that the drug responsiveness varies depending on the number of cells constituting the spheroid, and the drug responsiveness tends to be suitable in a case where the number of cells is low.

### [Experimental Example 10]

### (Examination of 3D Spheroid-Cultured Nerve Cells)

Spheroids having different numbers of cells were produced and diameters thereof were measured. In addition, various parameters shown in Table 1 below were calculated.

Human iPSC-derived nerve cells (Excitatory Neurons, Elixirgen Scientific, Inc.) and human-derived primary cultured astrocytes (ScienCell Research Laboratories, Inc.) were mixed so that the number of cells per well was 8,000 for the nerve cells and 8,000 for the astrocytes, 4,000 for the nerve cells and 4,000 for the astrocytes, 2,000 for the nerve cells and 2,000 for the astrocytes, 1,000 for the nerve cells and 1,000 for the astrocytes, 500 for the nerve cells and 500 for the astrocytes, and 250 nerve cells and 250 astrocytes, and seeded on a plate for producing a spheroid. As the culture medium, Neurobasal Plus (Thermo Fisher Scientific, Inc.) to which B-27 Plus Supplement (Thermo Fisher Scientific, Inc.), Glutamax (Thermo Fisher Scientific, Inc.), 200 µM ascorbic acid (FUJIFILM Wako Pure Chemical Corporation), and a 10% Neuron Culture medium (FUJIFILM Wako Pure Chemical Corporation) had been added was used. Subsequently, the cells were cultured for 5 weeks and an image thereof was captured with a phase-contrast microscope.

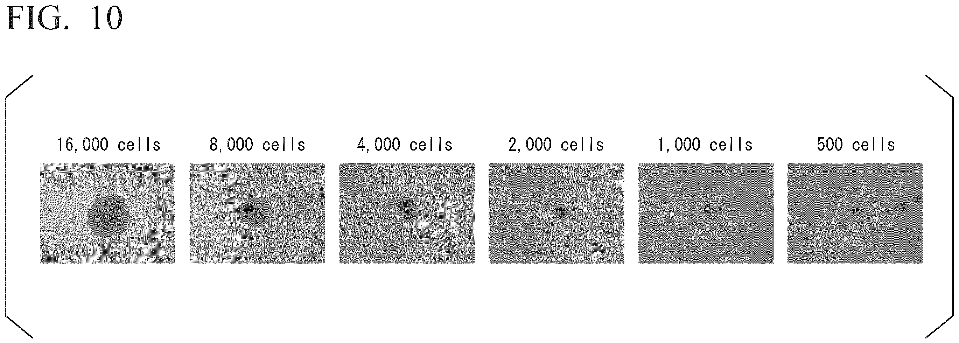

FIG. 10 is a representative image of the imaged spheroid. In FIG. 10, "16,000 cells" indicates a spheroid produced by mixing 8,000 nerve cells and 8,000 astrocytes, "8,000 cells" indicates a spheroid produced by mixing 4,000 nerve cells and 4,000 astrocytes, "4,000 cells" indicates a spheroid produced by mixing 2,000 nerve cells and 2,000 astrocytes, "2,000 cells" indicates a spheroid produced by mixing 1,000 nerve cells and 1,000 astrocytes, "1,000 cells" indicates a spheroid produced by mixing 500 nerve cells and 500 astrocytes, and "500 cells" indicates a spheroid produced by mixing 250 nerve cells and 250 astrocytes.

Table 1 below shows various parameters of each spheroid. Some of the parameters were calculated based on the diameter of the spheroid measured based on the image of FIG. 10. In Table 1 below, the "Surface area" means a surface area of the spheroid, the "Volume" means the volume of the spheroid, and the "Cell density" means a cell density of the spheroid. The "Radius of 10 µm inward" means a radius of a spheroid in a case where it is assumed that the diameter of one cell is 10 µm and one cell layer positioned at an outer edge of the spheroid is removed. The "Volume of 10 µm inward" means the volume of the spheroid in a case of assuming that the diameter of one cell is 10 µm and one cell layer positioned at an outer edge of the spheroid is removed. The "Volume of surface 10 µm" means the volume of one cell layer positioned at an outer edge of the spheroid, assuming that the diameter of one cell is 10 µm. The "Proportion (%) of volume of surface 10 µm to volume of entire spheroid" means the proportion (%) of the volume of one cell layer positioned at an outer edge of the spheroid, assuming that the diameter of one cell, is 10 µm to the volume of the entire spheroid. The "Number of cells present in volume of surface 10 µm" means the number of cells of one cell layer positioned at an outer edge of the spheroid, assuming that the diameter of one cell is 10 µm.

**[Table 1]**

| | | | | | | |
|---|---|---|---|---|---|---|
| Total number of cells (cells) | 16,000 | 8,000 | 4,000 | 2,000 | 1,000 | 500 |
| Radius of spheroid (µm) | 225 | 162.5 | 112.5 | 75 | 56.25 | 37.5 |
| Surface area (µm²) | 635,850 | 331,662.5 | 158,962.5 | 70,650 | 39,740.625 | 17,662.5 |
| Volume (µm³) | 47,688,750 | 17,965,052.1 | 5,961,093.75 | 1,766,250 | 745,136.719 | 220,781.25 |
| Cell density (cells/10³ µm³) | 0.336 | 0.445 | 0.671 | 1.132 | 1.342 | 2.265 |
| Radius of 10 µm inward (µm) | 215 | 152.5 | 102.5 | 65 | 46.25 | 27.5 |
| Volume of 10 µm inward (µm³) | 41,608,663 | 14,848,340 | 4,508,582 | 1,149,763 | 414,194 | 87,070 |
| Volume of surface 10 µm (µm³) | 6,080,087 | 3,116,712 | 1,452,512 | 616,487 | 330,943 | 133,712 |
| Proportion of volume of surface 10 µm to volume of entire spheroid (%) | 12.75 | 17.35 | 24.37 | 34.90 | 44.41 | 60.56 |
| Number of cells present in volume of surface 10 µm (cells) | 2,040 | 1,388 | 975 | 698 | 444 | 303 |

From the results of Experimental Examples 2 to 10, it was found that the spheroid having a total number of cells of 4,000 tended to exhibit the most suitable drug responsiveness, and the spheroid having a total number of cells of 8,000 and the spheroid having a total number of cells of 16,000 tended to exhibit more suitable drug responsiveness in this order. It is considered that the spheroid having a total number of cells of 2,000 or less also exhibits a drug responsiveness, but it is presumed that the drug responsiveness was not recognized since a detection sensitivity of the device used in the calcium assay was below the detection sensitivity of the spheroid.

In the present Experimental Examples, the results were that the drug sensitivity relatively decreased with the total number of cells of 8,000 compared to the total number of cells of 4,000, and further with the total number of cells of 16,000 compared to the total number of cells of 8,000. This is presumed to be because the number of cells is high and the size of the spheroid is large, the drug permeates only the surface of the spheroid, and the number of cells which are in contact with the drug and exhibit a drug response is smaller than the number of cells which are not in contact with the drug and do not exhibit a drug response (because the expected drug response is being obscured).

Therefore, in order to observe the drug response with high sensitivity, a state in which the drug can be in contact with the spheroid and the proportion of cells that exhibit the drug response is high is preferable. Therefore, the present inventors have focused on the surface of the spheroid, and have examined the "volume ratio of one cell layer" suitable for representing a ratio of cells present on the surface, and have therefore found suitable conditions for spheroids having excellent drug responsiveness.

In a case where the types of cells are different, the spheroid sizes may be different even in a case where the numbers of cells are the same. Therefore, it is necessary to specify an appropriate number (range) of cells for each cell to be used, in which the volume ratio of one cell layer is 15% to 30%.

Specifically, first, a plurality of spheroids in which the number of cells to be used is gradually increased (for example, the total number of cells is 20,000, 16,000, 12,000, 8,000, 4,000, 2,000, or the like) are formed. Then, "the volume ratio of one cell layer/the volume of the entire cell" for each cell number is calculated, based on Expression (1). Then, the number of cells (range) at which the value is 15% to 30% is calculated. Hereinafter, production of the spheroid is performed using a predetermined number of cells according to the type of cells to be used. In this manner, it is possible to produce a spheroid having suitable drug sensitivity (drug responsiveness).

The present invention includes the following aspects.
[1] A nervous system cell-containing spheroid including at least one kind of nerve cells and nervous system cells other than the nerve cells, in which the proportion of the volume of one cell layer positioned at an outer edge of the spheroid to the volume of the entire spheroid is 15% to 30%.
[2] The nervous system cell-containing spheroid according to [1], in which the nervous system cells other than the nerve cells include astrocytes.
[3] The nervous system cell-containing spheroid according to [2], in which the proportion of the number of the nerve cells to the number of the astrocytes (the number of the nerve cells/the number of the astrocytes) is 1.
[4] A method for producing a nervous system cell-containing spheroid, the method including a step of mixing at least one kind of nerve cells after cell lineage determination and nervous system cells other than the nerve cells at a predetermined proportion to form a spheroid, in which the proportion of the volume of one cell layer positioned at an outer edge of the spheroid to the volume of the entire spheroid is adjusted to 15% to 30% in the step.
[5] The production method according to [4], in which the nervous system cells other than the nerve cells include astrocytes.
[6] The production method according to [5], in which the proportion of the number of the nerve cells to the number of the astrocytes (the number of the nerve cells/the number of the astrocytes) is 1.
[7] A method for evaluating a test substance, the method including a step of incubating a nervous system cell-containing spheroid in the presence of the test substance, and a step of evaluating an effect of the test substance on the nervous system cell-containing spheroid, in which the nervous system cell-containing spheroid includes at least one kind of nerve cells and nervous system cells other than the nerve cells, and the proportion of the volume of one cell layer positioned at an outer edge of the spheroid to the volume of the entire spheroid is 15% to 30%.
[8] The evaluation method according to [7], in which the nervous system cells other than the nerve cells include astrocytes.
[9] The evaluation method according to [8], in which the proportion of the number of the nerve cells to the number of the astrocytes (the number of the nerve cells/the number of the astrocytes) is 1.

### Description of Related Art

### [Patent Document]

[Patent Document 1] Japanese Unexamined Patent Application, First Publication No. 2021-185906A

While preferred embodiments of the invention have been described and illustrated above, it should be understood that these are exemplary of the invention and are not to be considered as limiting. Additions, omissions, substitutions, and other modifications can be made without departing from the scope of the invention. Accordingly, the invention is not to be considered as being limited by the foregoing description and is only limited by the scope of the appended claims.

## Claims

1. A nervous system cell-containing spheroid comprising:
at least one kind of nerve cells and nervous system cells other than the nerve cells,
wherein a proportion of a volume of one cell layer positioned at an outer edge of the spheroid to a volume of the entire spheroid is 15% to 30%.

2. The nervous system cell-containing spheroid according to claim 1,
wherein the nervous system cells other than the nerve cells include astrocytes.

3. The nervous system cell-containing spheroid according to claim 2,
wherein a proportion of the number of the nerve cells to the number of the astrocytes (the number of the nerve cells/the number of the astrocytes) is 1.

4. A method for producing a nervous system cell-containing spheroid, the method comprising:
a step of mixing at least one kind of nerve cells after cell lineage determination and nervous system cells other than the nerve cells at a predetermined proportion to form a spheroid,
wherein a proportion of a volume of one cell layer positioned at an outer edge of the spheroid to a volume of the entire spheroid is adjusted to 15% to 30% in the step.

5. The production method according to claim 4,
wherein the nervous system cells other than the nerve cells include astrocytes.

6. The production method according to claim 5,
wherein a proportion of the number of the nerve cells to the number of the astrocytes (the number of the nerve cells/the number of the astrocytes) is 1.

7. A method for evaluating a test substance, the method comprising:
a step of incubating a nervous system cell-containing spheroid in a presence of the test substance; and
a step of evaluating an effect of the test substance on the nervous system cell-containing spheroid,
wherein the nervous system cell-containing spheroid includes at least one kind of nerve cells and nervous system cells other than the nerve cells, and a proportion of a volume of one cell layer positioned at an outer edge of the spheroid to a volume of the entire spheroid is 15% to 30%.

8. The evaluation method according to claim 7,
wherein the nervous system cells other than the nerve cells include astrocytes.

9. The evaluation method according to claim 8,
wherein a proportion of the number of the nerve cells to the number of the astrocytes (the number of the nerve cells/the number of the astrocytes) is 1.
